# EUROPEAN PATENT APPLICATION

(11) **EP 4 745 121 A1**
(43) Date of publication of application: **20.05.2026**
(21) Application number: 24213152.2
(22) Date of filing: 15.11.2024
(51) Int. Cl.: C07C 291/00, C07F 5/02

(54) **PROCESS FOR PREPARING OF ORGANIC CARBAMOYL AZIDES**

(71) Applicant: All4Labels Group GmbH, 22969 Witzhave (DE)
(72) Inventor: Pace, Vittorio, 10125 Torino (IT); Miele, Margherita, 10125 Torino (IT); Castoldi, Laura, 10125 Torino (IT)
(74) Representative: RGTH

(57) **Abstract**

The invention relates to a process for preparing an organic carbamoyl azide of general formula (I) 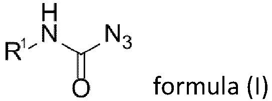 comprising the step of:
reacting a compound of general formula (II) 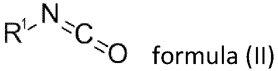 wherein R¹ is defined as herein, with an organic silyl azide in a suitable solvent in the presence of a Lewis base. The reactions are virtually quantitative in all cases, which can eliminate the need for lengthy and expensive purification steps. In addition, the reaction time is very short, resulting in a highly cost-effective process overall.

## Description

### TECHNICAL FIELD

The invention relates to a process for the production of organic carbamoyl azides.

### STATE OF THE ART

Functionalized amides constitute interesting and highly regarded chemical functionalities in materials science. Particularly, linking the amide sp² carbon to an azide group results in the formation of a special group referred to as carbamoyl azide, which exhibits an inherent chemical versatility due to the wide range of transformations possible for both the amide and azide groups. Despite these particular advantages and high relevance, the methods that enable the construction of the functionality are rather non straightforward and usually very complex, mainly due to the requirement to carry out at least two or three steps of chemical reactions.

The complexity of the known production methods is related to the structure and reactivity of the azide group. Organic azides are organic compounds with a chemical group known as azide (N₃). The general formula of organic azides - in which delocalisation effects are indicated - could be represented as follows: 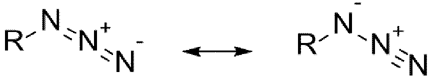 whereby R is an organic radical. Organic azides, such as methyl azide, often decompose explosively. Accordingly, their synthesis is associated with serious safety issues, including limited sustainability.

As a matter of fact, the so-called Curtius rearrangement, carried out on acyl azides, is still one of the most reliable routes to the title compounds. Alternatively, the azidation of aldehydes has also been proposed as an indirect protocol for preparing carbamoyl azides.

Not only the necessity of multi-step procedures, in which each individual intermediate step requires the proper purification of the involved intermediate products, whereby the yields drop significantly, but also the manipulation of toxic chemicals, are in sharp contrast to a sustainable approach in terms of environmental and health pollution with alternative, environmentally friendly reaction media, while at the same time achieving higher reaction rates and lower reaction temperatures (12 principles of green chemistry by Paul T. Anastas and John C. Warner). It would therefore be desirable if the manipulation of chemical compounds associated with a high risk of explosion - such as compounds with azido groups - in experimental synthesis processes with high yields could be achieved in which the dangerous chemical reaction group could be used in a stable and thus safe form that retains its reactivity.

From the prior art, a number of proposals for the preparation of carbamoyl azides have been known, some of which will be discussed below:
For example, KR 10-0441 764 B1 describes vinyl carbamoyl azide derivatives of general formula 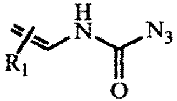 wherein R1 is a furyl group or a phenyl group, each optionally having one or more substituents selected from the group consisting of one or more C₁₋₆ alkyl, a halogen element, a C₁₋₆ alkoxy group, and a nitro group. The vinyl carbamoyl azide derivative is described to be more stable than a conventional carbamoyl azide derivative and may be used as an effective ingredient in a composition for sterilizing agricultural pathogens. The vinyl carbamoyl azide derivative represented by the formula 1 is produced by reacting an isocyanate compound (R₁NCO) and hydrazoic acid (HN₃) or sodium azide (NaN₃).

AU 531 375 B2 discloses N-alkyl-N-nitrosocarbamoyl azides. The process for the preparation of an N-(C₂-C₆)-alkyl-N-nitrosocarbamoyl azide or an N-halo-(C₂-C₆)-alkyl-N-nitrosocarbamoyl azide comprises the reaction of an N-(C₂-C₆)-alkyl carbamoyl azide or an N-halo-(C₂-C₆)-alkyl carbamoyl azide with nitrogen tetroxide at a temperature within the range of 0 to -10°C in a solvent which under the reaction conditions is inert to the reactants. According to a second aspect, the process is performed by reacting a (C₂-C₆) alkyl isocyanate or a halo(C₂-C₆) alkyl isocyanate with an azide to form an N-(C₂-C₆) alkyl carbamoyl azide or an N-halo(C₂-C₆) alkyl carbamoyl azide and reacting the N(C₂-C₆) alkyl carbamoyl azide or the N-halo(C₂-C₆)-alkyl carbamoyl azide with nitrogen tetroxide in the cold in a solvent which under the reaction conditions is inert to the reactants.

Furthermore, DE 26 59 862 B2 is directed to N-(2-chloroethyl)-N-nitrosocarbamoyl azide Cl-CH₂-CH₂-N(NO)CON₃ and the manufacturing thereof. The production of N-(2-chloroethyl)-N-nitroso-carbamoyl azide is carried out by reacting 2-chloroethyl-isocyanate in an inert solvent with activated sodium azide with the addition of hydrochloric acid and the formed 2-chloroethyl carbamoyl azide is reacted with nitrogen tetroxide in an inert solvent at about 0 °C to obtain N-(2-chloroethyl)-N-nitroso-carbamoyl azide.

In addition, DE 26 23 420 B1 describes a process for producing unsymmetrical 1,3-disubstituted nitrosoureas. In the process for the production of 1,3-disubstituted nitrosoureas an alkali metal isocyanate is reacted with an alkali metal azide in the cold, the resulting alkylcarbamoyl azide is nitrosated with N₂O₄ in the cold and then the resulting N-alkyl-N-nitroso-carbamoyl azide is reacted with a diamine, amino alcohol or amino acid derivative in a solvent that is inert to the reactants.

The disadvantages of the prior art are that the carbamoyl azides are produced using methods that in some cases require particularly toxic and hazardous reagents and particularly toxic solvents, take a long time to react and also require several process steps to produce and, if necessary, functionalise the carbamoyl azides. In some cases, low yields are achieved because several process steps have to be carried out and the intermediate products obtained have to be subjected to a purification process in each case.

It is therefore an object of the present invention to provide a method for the production of organic carbamoyl azides and overcome disadvantages of the prior art while providing the end products with as few process steps as possible. In addition, the manufacturing process should be as simple as possible to carry out and provide the highest possible yields.

### SUMMARY OF THE INVENTION

The above-described problem is solved according to the present invention by a process for preparing a compound of general formula (I) 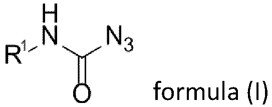
wherein R¹ is selected from the group consisting of
C₁-C₁₈-alkyl, which is optionally substituted with one or more substituents R⁴;
C₄-C₁₁-heterocyclyl, which is optionally substituted with one or more substituents R⁴;
C₂-C₁₈-alkenyl, which is optionally substituted with one or more substituents R⁴;
C₂-C₁₈-alkynyl, which is optionally substituted with one or more substituents R⁴;
C₆-C₁₈-aryl, which is optionally substituted with one or more substituents R⁴;
C₃-C₁₇-heteroaryl, which is optionally substituted with one or more substituents R⁴;
R⁴ is at each occurrence independently from another selected from the group consisting of:
   NO₂,
   CF₃,
   CN,
   OH,
   =O,
   OR⁵,
   -(C=O)R⁵,
   -(C=O)OR⁵,
   halogen,
   C₁-C₁₂-alkyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
   C₄-C₁₁-heterocyclyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
   C₂-C₁₂-alkenyl, which is a linear, branched or cyclic alkenyl group, optionally mono- or polysubstituted, the substituents being selected from the group consisting of C₆-C₁₀-aryl, NO₂, F₃CO, CF₃, CN, OH, =O, OR⁵, -(C=O)R⁵, -(C=O)OR⁵, halogen;
   C₂-C₁₂-alkynyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
   C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, OH, =O, OR⁵, -(C=O)R⁵, -(C=O)OR⁵, halogen, wherein C₁-C₅-alkyl is optionally substituted with one or more OH groups, preferably one OH group, and C₂-C₈-alkenyl is optionally substituted with one or more OH groups, preferably one OH group;
   C₃-C₁₇-heteroaryl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
   R⁵ is at each occurrence independently from another selected from the group consisting of:
      CF₃,
      C₁-C₁₂-alkyl, which is optionally mono- or polysubstituted, the substituents being selected from the group consisting of C₁-C₅-alkyl, NO₂, F₃CO, CF₃, CN, halogen; and
      C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups, preferably one OH group, and C₂-C₈-alkenyl is optionally substituted with one or more OH groups, preferably one OH group;
      comprising the step of:
         reacting a compound of general formula (II) 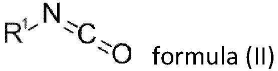
         wherein R¹ is defined as hereinbefore,
         with an organic silyl azide,
         in a suitable solvent in the presence of a Lewis base.

The present invention is also directed to a process for preparing a compound of general formula (Ia) 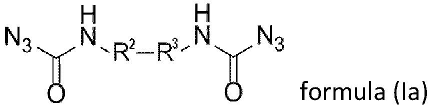
wherein R² and R³ are the same or different and are independently selected from the group consisting of
C₁-C₁₈-alkyl, which is optionally substituted with one or more substituents R⁴;
C₄-C₁₂-heterocyclyl, which is optionally substituted with one or more substituents R⁴;
C₂-C₁₈-alkenyl, which is optionally substituted with one or more substituents R⁴;
C₂-C₁₈-alkynyl, which is optionally substituted with one or more substituents R⁴;
C₆-C₁₈-aryl, which is optionally substituted with one or more substituents R⁴;
C₃-C₁₇-heteroaryl, which is optionally substituted with one or more substituents R⁴;
R⁴ is at each occurrence independently from another selected from the group consisting of:
   NO₂,
   CF₃,
   CN,
   OH,
   =O,
   OR⁵,
   -(C=O)R⁵,
   -(C=O)OR⁵,
   halogen,
   C₁-C₁₂-alkyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
   C₄-C₁₁-heterocyclyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
   C₂-C₁₂-alkenyl, which is a linear, branched or cyclic alkenyl group, optionally mono- or polysubstituted, the substituents being selected from the group consisting of C₆-C₁₀-aryl, NO₂, F₃CO, CF₃, CN, OH, =O, OR⁵, -(C=O)R⁵, -(C=O)OR⁵, halogen;
   C₂-C₁₂-alkynyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
   C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, OH, =O, OR⁵, -(C=O)R⁵, -(C=O)OR⁵, halogen, wherein C₁-C₅-alkyl is optionally substituted with one or more OH groups, preferably one OH group, and C₂-C₈-alkenyl is optionally substituted with one or more OH groups, preferably one OH group;
   C₃-C₁₇-heteroaryl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
   R⁵ is at each occurrence independently from another selected from the group consisting of:
      CF₃,
      C₁-C₁₂-alkyl, which is optionally mono- or polysubstituted, the substituents being selected from the group consisting of C₁-C₅-alkyl, NO₂, F₃CO, CF₃, CN, halogen; and
      C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups, preferably one OH group, and C₂-C₈-alkenyl is optionally substituted with one or more OH groups, preferably one OH group;
      whereby one of R² or R³ may also represent a direct bond,
      comprising the step of:
         reacting a compound of general formula (Ila) 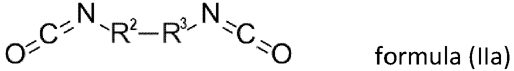
         wherein R² and R³ are defined as hereinbefore,
         with an organic silyl azide,
         in a suitable solvent in the presence of a Lewis base.

It is a single-step process that enables the direct conversion of isocyanates into carbamoyl azides by reaction with organic azides in a previously unknown manner.

### DETAILED DESCRIPTION OF THE INVENTION

### DEFINITION OF GENERAL TERMS

Terms not specifically defined herein should be given the meaning which a person skilled in the art would give them in light of the disclosure and context.

For all compounds disclosed herein, in the event the nomenclature is in conflict with the structure, it shall be understood that the compound is defined by the structure.

The compounds of the invention are only those which are contemplated to be 'chemically stable' as will be appreciated by those skilled in the art. For example, a compound which would have a 'dangling valency', or a 'carbanion' are not compounds contemplated by the inventive methods disclosed herein.

Unless otherwise indicated, the chemical formula or chemical name in the entire description and the appended claims include all possible isomers, including tautomers and all stereo, optical and geometric isomers (e.g. enantiomers, diastereomers, etc.) and their racemates.

As used above and herein, the term 'alkyl group' may be understood in the broadest sense and denotes, either alone or in combination with another radical, an acyclic or cyclic, saturated, branched or linear hydrocarbon radical having 1 to n carbon atoms, where n represents an integer which represents the stated number of carbon atoms. For example, the term alkyl comprises the substituents such as methyl (Me), ethyl (Et), n-propyl (ⁿPr), i-propyl ('Pr), cyclopropyl, n-butyl (ⁿBu), i-butyl ('Bu), s-butyl (^{s}Bu), t-butyl (^{t}Bu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neo-hexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl, etc.

The term 'cycloalkyl' is intended to be included within the term 'alkyl' and denotes, either alone or in combination with another radical, a cyclic, saturatedhydrocarbon radical with 3 to n C atoms, where n represents an integer representing the stated number of carbon atoms. The cyclic group may be mono-, bi-, tri- or spirocyclic, most preferably monocyclic which can be bound via any position of the cycloalkyl group. Examples of such cycloalkyl groups include cyclopropyl, cyclobutyl, cyclopentyl, 1-methylcyclopentyl, cyclohexyl, cycloheptyl, 1-methylcyclohexyl, n-octyl, 2-ethylhexyl, cyclooctyl, 1-bicyclo[2,2,2]octyl, 2-bicyclo[2,2,2]-octyl, bicyclo[3.2.1.]octyl, Cyclononyl, Cyclododecyl, spiro[4.5]decyl, norpinyl, norbonyl, norcaryl, adamantyl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl and 1-(n-decyl)-cyclohex-1-yl, etc.

As used herein, the term 'alkenyl', either alone or in combination with another radical, comprises linear, branched, and cyclic alkenyl substituents. The term alkenyl group exemplarily comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl, etc.

As used herein, the term 'alkynyl', either alone or in combination with another radical, comprises linear, branched, and cyclic alkynyl substituents. The term alkynyl group exemplarily comprises ethynyl, propynyl (propargyl), butynyl, pentynyl, hexynyl, heptynyl or octynyl.

All alkyl, alkenyl and alkynyl groups shall be understood as being branched or unbranched where structurally possible and unless otherwise specified.

As used herein, the residue -OR⁵ is understood to be an alkoxy group, either alone or in combination with another radical, which comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy, 2-methylbutoxy, phenoxy, naphthoxy, etc.

As used herein, the residue -(C=O)R⁵ is understood to be an acyl group, either alone or in combination with another radical, which comprises linear, branched, and cyclic acyl substituents. The term acyl group exemplarily comprises acetyl, benzoyl- or pyridinoyl group etc.

As used herein, the residue -(C=O)OR⁵, is understood to represent an alkyloxycarbonyl or aryloxycarbonyl group, which is synonymous and interchangeable with a carboxylic ester group, either alone or in combination with another radical, encompassing linear, branched and cyclic carboxylic ester substituents. For example, the term 'carboxylic ester group' includes methyl acetate, ethyl acetate, butyl acetate, gamma-butyrolactone, etc.

As used herein, the term 'heterocyclyl' is understood to respresent a stable nonaromatic 4 to 8 membered monocyclic heterocyclic radical or a stable nonaromatic 6 to 11-membered fused bicyclic, bridged bicyclic or spirocyclic heterocyclic radical which can be bound via any position of the heterocyclyl group. The 6 to 11 membered heterocycle consists of carbon atoms and one or more, preferably from one to four heteroatoms chosen from nitrogen, oxygen and sulfur. The heterocycle may be either saturated or partially unsaturated. Non-limiting examples of nonaromatic 4 to 8 membered monocyclic heterocyclic radicals include or are derived from tetrahydrofuranyl, azetidinyl, pyrrolidinyl, pyranyl, tetrahydropyranyl, dioxanyl, dioxaborolan, such as 4,4,5,5-tetramethyl-1,3,2-dioxaborolan, thiomorpholinyl, 1,1-dioxo-lamda⁶-thiomorpholinyl, morpholinyl, imidazolinyl, tetrahydrothiophenyl, piperidinyl, piperazinyl, and azepinyl. Non-limiting examples of nonaromatic 6 to 11-membered fused bicyclic radicals include octahydroindolyl, octahydrobenzofuranyl, and octahydrobenzothiophenyl. Non-limiting examples of nonaromatic 6 to 11-membered bridged bicyclic radicals include 2-azabicyclo[2.2.1]heptanyl, 3-azabicyclo[3.1.0]hexanyl, and 3-azabicyclo[3 .2.1]octanyl. Non-limiting examples of nonaromatic 6 to 11-membered spirocyclic heterocyclic radicals include 7-aza-spiro[3,3]heptanyl, 7-spiro[3,4]octanyl, and 7-aza-spiro[3,4]octanyl. The term 'heterocyclyl' is intended to include all possible isomeric forms.

As used throughout the present invention, the term 'aryl' may be understood in the broadest sense as any mono-, bi-, tri- or tetracyclic aromatic moieties. In the present invention, an aryl group contains 6 to 18 ring atoms. The term 'aryl' as used herein, either alone or in combination with another radical, denotes a carbocyclic aromatic monocyclic group containing 6 carbon atoms which may be further fused to at least a second 4 or 5- or 6-membered carbocyclic group which may be aromatic, saturated or unsaturated. Therefore, the term 'aryl' also includes partially hydrogenated aromatic systems, such as 1,2,3,4-tetrahydronaphthalene.

As used herein, the term 'heteroaryl' may be understood in the broadest sense and may, for example, contain mono-, bi-, tri- or tetracyclic heteroaromatic moieties, that include at least one heteroatom. In particular, a heteroaryl group contains 3 to 17 aromatic ring atoms, of which at least one is a heteroatom. The term 'heteroatom' as used herein shall be understood to mean atoms other than carbon such as O, N, S and P. In particular, the heteroaromatic ring includes one to four heteroatoms. According to the invention, a condensed (annulated) aromatic or polycycle is built of two or more single aromatic, e.g. formed via a condensation reaction. The term 'heteroaryl' is intended to include all possible isomeric forms.

In particular, the term aryl group or heteroaryl group comprises groups which can be bound via any position of the aryl or heteroaryl group, and are, for example, derived from benzene (phenyl), benzyl, tolyl, naphthalene (naphthyl), 1,2,3,4-tetrahydronaphthalene, anthracene, phenanthrene, pyrene, dihydropyrene, chrysene, fluoranthene, benzanthracene, benzphenanthrene, tetracene, furan, benzofuran, isobenzofuran, dibenzofuran, dihydrobenzofuran, benzopyran, thiophene, thienyl, benzothiophene, isobenzothiophene, dibenzothiophene; pyrrole, indole, isoindole, carbazole, tetrazol, pyridine, quinoline, isoquinoline, acridine, phenanthridine, benzo-5,6-quinoline, benzo-6,7-quinoline, benzo-7,8-quinoline, dihydro-2H-quinolinyl, tetrahydroquinolinyl, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, thieno[2,3-d]pyrimidinyl, 1,3,5-triazine, quinazolin, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine, thiadiazol, benzodioxolyl and benzothiadiazole or combinations of the abovementioned groups.

As used above and herein, the terms 'halogen' and 'halo' may be understood in the broadest sense as being preferably fluorine, chlorine, bromine or iodine. The term 'halogenated' or 'substituted with halogen' comprises 'partially or fully halogenated' and includes, for example, mono-, di- or tri-halo derivatives at one or more carbon atoms. For alkyl, an example would be -CH₂CHF₂ or -CF₃.

The expressions "comprising", "comprise", "comprised", "containing", "contain" or "contained" shall also encompass the more specific term "consisting of" unless otherwise stated or apparent from the context.

In addition, it should be noted that in this disclosure, the singular and plural forms are not used in a restrictive manner. As used herein, the singular forms "a", "an", "one" and "the" therefore refer to both the singular and the plural, unless otherwise stated or apparent from the context.

### DETAILED DESCRIPTION OF THE INVENTION

The process according to the invention is a one-step process that enables the direct conversion of isocyanates into carbamoyl azides by the reaction with organic silyl azides. The general reaction equations are as follows:

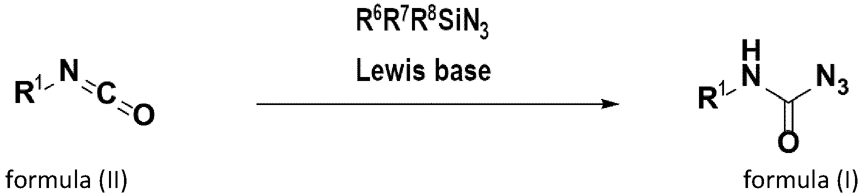

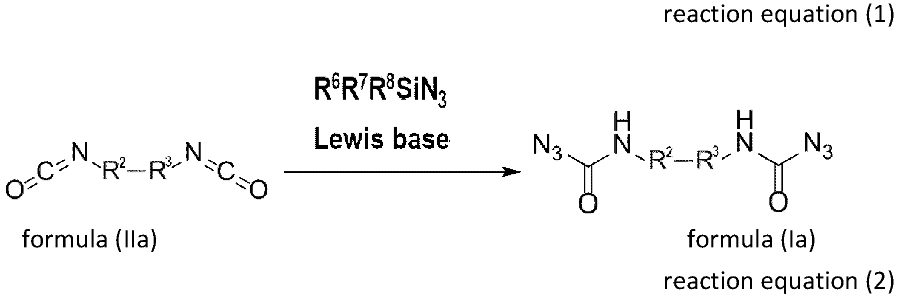

R¹, R², R³, R⁶, R⁷ and R⁸ are selected as defined herein.

The reaction equation (1) shows the conversion of an isocyanate compound into a carbamoyl azide compound. The reaction equation (2) shows the conversion of a diisocyanate compound into a dicarbamoyl azide compound. In the formulas (Ia) and (Ila) one of R² or R³ may also represent a direct bond so that an OCN-R²-NCO or OCN-R³-NCO unit in formula (Ila) or a N₃ONH-R²-NHON₃ or N₃ONH-R³-NHON₃ unit in formula (Ia) results.

This concept enables the direct production of the title compounds in a single synthetic step.

The process according to the invention involves the release of azido anions from an organic silyl azide in the presence of isocyanates, and is an extremely simple and conceptually easy-to-understand synthesis of carbamoyl azides that can be easily implemented in practice. The starting materials, both organic silyl azides and isocyanates, are commercially available in a highly reactive and cost-effective form. In addition, organic silyl azides are easy-to-handle reagents.

After numerous attempts at carefully optimising the reaction, it is assumed, but not bound by theory, that there exist three influences which are of interest for the conversion: the stoichiometry, the Lewis base for activating the organic azide and the chosen reaction conditions, such as temperature, solvent and reaction time. It was found that the process could be applied to any type of isocyanate without any problems, which indicated that the process is generally valid and robust. In addition, the process could be readily validated by experiments, i.e. the end products could be clearly verified and documented, demonstrating that the process is suitable and works as expected in any case.

In an unexpected way, chiral carbamoyl azides can be readily prepared according to the invention. The use of enantiomeric isocyanates as starting products was associated with the complete preservation of stereochemistry in the end product, resulting in enantiomerically pure end products (enantiomeric excess ee > 98%, in particular ee > 99 %)). This is very remarkable, since the preparation of chiral carbamoyl azides using the known methods from the prior art has so far been very difficult and almost never free of racemisation.

The starting compounds in the form of isocyanates are not further limited in the context of the invention. Any isocyanate can be used. These are, for example, aliphatic, aromatic or heteroaromatic isocyanates, which can have a variety of different substituents. The isocyanate compound contains one NCO group or two NCO groups which are then each converted to the azide.

The isocyanates have the general formula (II) 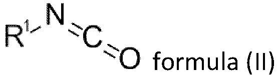 wherein R¹ is selected as defined above.

Alternatively, the isocyanate may also have the general formula (Ila) 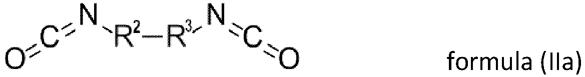 wherein R² and R³ are as defined above. The compound of formula (Ila) represents the dimer of the isocyanate compound of formula (II), wherein there are 2 azide groups. However, there may be more than 2 azide groups present in the isocyanate compound as already mentioned.

According to a preferred embodiment, R¹, R² and R³ are independently selected from the group consisting of
C₁-C₁₀-alkyl, which is a linear, branched or cyclic alkyl group, optionally substituted with one or more substituents R⁴, particularly preferred are methyl (Me), ethyl (Et), n-propyl (ⁿPr), i-propyl (ⁱPr), cyclopropyl, n-butyl (ⁿBu), i-butyl ('Bu), s-butyl (^{s}Bu), t-butyl (^{t}Bu), cyclobutyl, 2-methylbutyl, n-pentyl, s-pentyl, t-pentyl, 2-pentyl, neo-pentyl, cyclopentyl, n-hexyl, adamantyl (Ad), cyclohexyl (Cy), 4-methylcyclohexyl, cyclooctyl, 3,3,5-trimethylcyclohexyl, 1-(trifluoromethyl)cyclopropyl, tetrahydronaphthyl (1,4,5,8-tetrahydronapthyl);
C₄-C₁₁-heterocyclyl, which is optionally substituted with one or more substituents R⁴, particularly preferred are tetrahydrofuranyl, azetidinyl, pyrrolidinyl, pyranyl, tetrahydropyranyl, dioxanyl, dioxaborolan morpholinyl, piperidinyl, piperazinyl, azepinyl, imidazolinyl, tetrahydrothiophenyl;
C₂-C₈-alkenyl, which is a linear, branched or cyclic alkenyl group, optionally substituted with one or more substituents R⁴, particularly preferred are allyl, isopropenyl, ethenyl (vinyl), isophorone, styryl;
C₂-C₈-alkynyl, which is optionally substituted with one or more substituents R⁴, particularly preferred are butinyl, ethynyl, propargyl, 3-methylbut-1-inyl, ;
C₆-C₁₄-aryl, which is optionally substituted with one or more substituents R⁴, particularly preferred are phenyl, benzyl, phenethyl, tolyl, biphenyl, naphthyl, tetrahydronapthyl (1,2,3,4-tetrahydronapthyl) , anthracene, cinnamyl, vinylphenyl, ethynylphenyl; and
C₃-C₁₇-heteroaryl, which is optionally substituted with one or more substituents R⁴, particularly preferred are furan, benzofuran, benzopyran, thiophene, thienyl, benzothiophene, dibenzothiophene; pyrrole, indole, carbazole, tetrazol, pyridine, quinoline, isoquinoline, tetrahydroquinolinyl, pyrazole, imidazole, benzimidazole, oxazole, benzoxazole, isoxazole, thiazole, pyrimidine, benzopyrimidine, quinazolin, pyrazine, phenazine, phenanthroline, benzothiadiazole.

The expression 'one or more substituents R⁴' is understood to mean one, two, three, four or more substituents wherever this is possible.

According to a preferred embodiment, R¹, R² and R³ are independently selected from the group consisting of unsubstituted phenyl, phenyl substituted with one or more R⁴, unsubstituted naphthyl, naphthyl substituted with one or more R⁴, unsubstituted cyclohexane or cyclohexane substituted with one or more R⁴, whereby the substituents are selected from the group consisting of C₁-C₅-alkyl, C₂-C₈-alkenyl, NO₂, F₃CO, CF₃, CN or halogen, wherein C₁-C₅-alkyl is optionally substituted with one or more OH groups and C₂-C₈-alkenyl is optionally substituted with one or more OH groups.

According to another embodiment, when R¹ is selected from the group consisting of phenyl substituted with one or more R⁴, naphthyl substituted with one or more R⁴, or cyclohexane substituted with one or more R⁴, the one or more R⁴ substituents are preferably present in the ortho and/or para position to the isocyanate group of formula (II) or carbamoyl azide group of formula (I), where possible. Preferably, the substituents are selected from the group consisting of C₁-C₅-alkyl, C₂-C₈-alkenyl, NO₂, F₃CO, CF₃, CN or halogen, wherein C₁-C₅-alkyl is optionally substituted with one or more OH groups and C₂-C₈-alkenyl is optionally substituted with one or more OH groups.

This embodiment is explained in the following example:

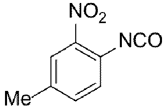

The isocyanate compound of formula (II) in the example case is a 4-methyl-2-nitrophenyl isocyanate. R¹ is a substituted phenyl wherein two R⁴ substituents are present. The two R⁴ substituents are in ortho and para position to the isocyanate group. The nitro group is ortho to the isocyanate group on the phenyl and the methyl group is in the para position to the isocyanate group on the phenyl. This is a preferred configuration when there are multiple substituents present.

The expression 'one or more R⁴' is understood to mean one, two, three, four or more substituents wherever this is possible.

Furthermore, according to another preferred embodiment, R⁴ is at each occurrence independently from another selected from the group consisting of:
NO₂,
F₃CO,
CF₃,
CN,
OH,
=O,
OR⁵,
-(C=O)R⁵,
-(C=O)OR⁵,
halogen,
C₁-C₅-alkyl, which is a linear, branched or cyclic alkyl group, which is optionally mono- or polysubstituted, the substituents being selected from the group consisting of NO₂, F₃CO, CF₃, CN, OH, =O, OR⁵, -(C=O)R⁵, -(C=O)OR⁵, halogen; particularly preferred are methyl (Me), ethyl (Et), n-propyl (ⁿPr), i-propyl (ⁱPr), cyclopropyl, n-butyl (ⁿBu), i-butyl (ⁱBu), s-butyl (^{s}Bu), t-butyl (^{t}Bu), cyclobutyl, 2-methylbutyl, n-pentyl, s-pentyl, t-pentyl, 2-pentyl, neo-pentyl, cyclopentyl;
C₄-C₁₁-heterocyclyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen; particularly preferred are tetrahydrofuranyl, azetidinyl, pyrrolidinyl, pyranyl, tetrahydropyranyl, dioxanyl, dioxaborolan morpholinyl, piperidinyl, piperazinyl, azepinyl, imidazolinyl;
C₂-C₈-alkenyl, which is a linear, branched or cyclic alkenyl group, optionally mono- or polysubstituted, the substituents being selected from the group consisting of C₆-C₁₀-aryl, NO₂, F₃CO, CF₃, CN, OH, =O, OR⁵, -(C=O)R⁵, -(C=O)OR⁵, halogen; particularly preferred are allyl, isopropenyl, ethenyl (vinyl), stiryl, ;
C₂-C₅-alkynyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen; particularly preferred are butinyl, ethynyl, propargyl, 3-methylbut-1-inyl;
C₆-C₁₄-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, OH, =O, OR⁵, -(C=O)R⁵, -(C=O)OR⁵, halogen, wherein C₁-C₅-alkyl is optionally substituted with one or more OH groups, preferably one OH group, and C₂-C₈-alkenyl is optionally substituted with one or more OH groups, preferably one OH group; particularly preferred are phenyl, benzyl, phenethyl, tolyl, biphenyl, naphthyl, anthracene, cinnamyl, vinylphenyl, ethynylphenyl; and
C₃-C₁₇-heteroaryl, which is optionally mono- or polysubstituted, the substituents being selected from the group consisting of NO₂, F₃CO, CF₃, CN, OH, =O, OR⁵, -(C=O)R⁵, -(C=O)OR⁵, halogen; particularly preferred are furan, benzofuran, benzopyran, thiophene, thienyl, benzothiophene, dibenzothiophene; pyrrole, indole, carbazole, tetrazol, pyridine, quinoline, isoquinoline, tetrahydroquinolinyl, pyrazole, imidazole, benzimidazole, oxazole, benzoxazole, isoxazole, thiazole, pyrimidine, benzopyrimidine, quinazolin, pyrazine, phenazine, phenanthroline, benzothiadiazole.

In the present disclosure, the expression 'mono- or polysubstituted' is understood to mean one, two, three, four or more substituents wherever this is possible.

According to another preferred embodiment R⁵ is at each occurrence independently from another selected from the group consisting of:
CF₃,
C₁-C₁₀-alkyl, which is a linear, branched or cyclic alkyl group, which is optionally mono- or polysubstituted, the substituents being selected from the group consisting of C₁-C₅-alkyl, NO₂, F₃CO, CF₃, CN, halogen, particularly preferred are methyl (Me), ethyl (Et), n-propyl (ⁿPr), i-propyl (ⁱPr), cyclopropyl, n-butyl (ⁿBu), i-butyl ('Bu), s-butyl (^{s}Bu), t-butyl (^{t}Bu), cyclobutyl, 2-methylbutyl, n-pentyl, s-pentyl, t-pentyl, 2-pentyl, neo-pentyl, cyclopentyl, n-hexyl, adamantyl (Ad), cyclohexyl (Cy), 4-methylcyclohexyl;
C₆-C₁₄-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups, preferably one OH group, and C₂-C₈-alkenyl is optionally substituted with one or more OH groups, preferably one OH group; particularly preferred are phenyl, benzyl, phenethyl, tolyl, biphenyl, naphthyl, anthracene, cinnamyl, vinylphenyl, ethynylphenyl.

The following are examples of aromatic isocyanate compounds that can be used as starting materials:

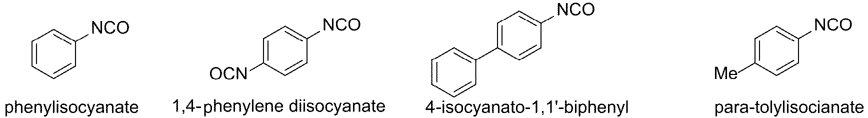

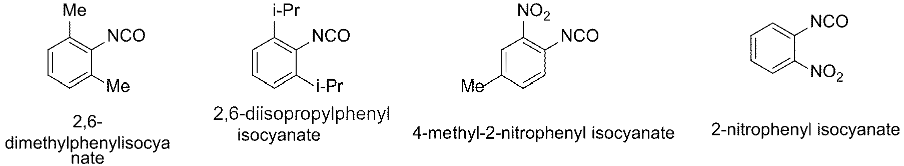

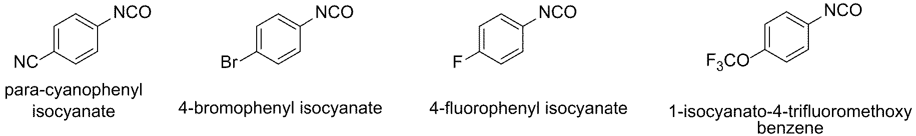

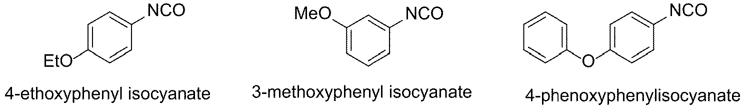

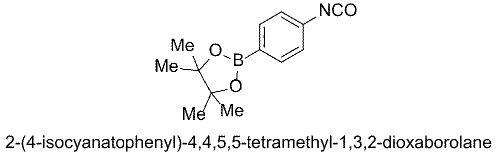

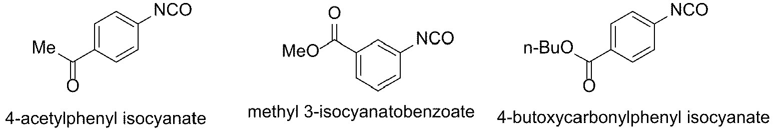

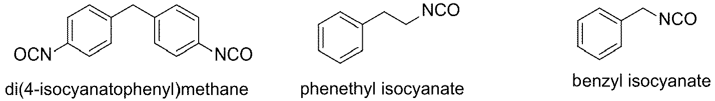

The following are some examples of aliphatic isocyanate compounds that can be used as starting materials:

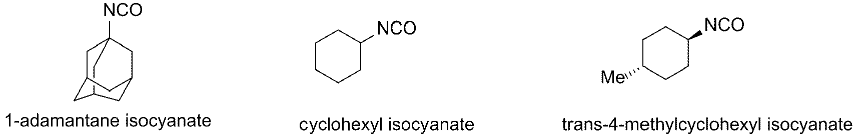

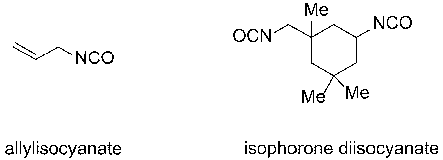

The present invention is, of course, not limited to the isocyanate compounds mentioned. Numerous other compounds can be used.

The other starting compound in the reaction to provide a carbamoyl aziode is an organic silyl azide. Silyl azides are compounds in which an azide group is bound to a silicon atom, which considerably improves the experimental manipulability of azides as chemical reagents. This is probably because the reactivity of the azide group as a nucleophile is drastically reduced. Therefore, although silyl azides are practical storage forms for the N₃ group, they are relatively inert synthons that must be activated in a preparative procedure before they can be used.

The term 'organic silyl azide' means that only organic groups, in particular only unsubstituted or substituted alkyl groups and/or unsubstituted or substituted aryl groups, are attached to the silicon atom. The organic silazyl azide is therefore selected in particular from an alkyl silazide, an aryl silazide, or an alkyl aryl silazide.

According to the invention, organic silyl azides are used, i.e. in particular silyl azides with the general formula (R⁶R⁷R⁸)SiN₃, in which the residues R⁶, R⁷, and R⁸ each represent organic residues, in particular alkyl and/or aryl groups, which may optionally each be substituted. A well-known silyl azide is trimethylsilyl azide, which can be readily obtained from standard chemical suppliers.

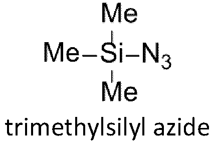

According to a preferred embodiment, the silyl azide comprises or consists of the general formula (III) as follows: 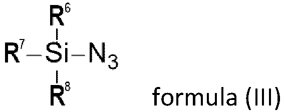
wherein R⁶, R⁷, and R⁸ are independently selected from the group consisting of
C₁-C₁₀-alkyl, which is optionally mono- or polysubstituted, the substituents being selected from the group consisting of C₁-C₅-alkyl, NO₂, F₃CO, CF₃, CN, halogen; and
C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups, preferably one OH group, and C₂-C₈-alkenyl is optionally substituted with one or more OH groups, preferably one OH group.

According to a preferred embodiment R⁶, R⁷, and R⁸ are independently selected from the group consisting of
C₁-C₅-alkyl, which is optionally mono- or polysubstituted, the substituents being selected from the group consisting of C₁-C₅-alkyl, NO₂, F₃CO, CF₃, CN, halogen; particularly preferred are methyl (Me), ethyl (Et), n-propyl (nPr), i-propyl ('Pr), cyclopropyl, n-butyl (ⁿBu), i-butyl ('Bu), s-butyl (^{s}Bu), t-butyl (^{t}Bu), cyclobutyl, 2-methylbutyl, n-pentyl, s-pentyl, t-pentyl, 2-pentyl, neo-pentyl, cyclopentyl, ; and
C₆-C₁₀-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups, preferably one OH group, and C₂-C₈-alkenyl is optionally substituted with one or more OH groups, preferably one OH group; particularly preferred are phenyl, benzyl, phenethyl, tolyl, biphenyl, naphthyl, cinnamyl, vinylphenyl, ethynylphenyl,.

According to another preferred embodiment R⁶, R⁷, and R⁸ are independently selected from the group consisting of
C₁-C₅-alkyl, which is optionally mono- or polysubstituted, the substituents being selected from the group consisting of NO₂, F₃CO, CF₃, CN, halogen; and
phenyl, which is optionally mono- or polysubstituted, the substituents being selected from the group consisting of C₁-C₅-alkyl, C₂-C₈-alkenyl, NO₂, F₃CO, CF₃, CN, halogen, wherein C₁-C₅-alkyl is optionally substituted with one or more OH groups, preferably one OH group, and C₂-C₈-alkenyl is optionally substituted with one or more OH groups preferably one OH group.

The Lewis bases used in the present process for the preparation of carbamoyl azides serve as activators for the organic silyl azide. The Lewis bases are not further limited according to the invention. Strong Lewis bases are preferred, but medium or weak Lewis bases can also be used. The following Lewis bases are mentioned by way of example: tetrabutylammonium fluoride, caesium fluoride, potassium fluoride, potassium-tert-butoxide and sodium methoxide. Other Lewis bases known to those skilled in the art may also be used, provided that they do not react in an undesired manner with the starting compounds and end products. It is also possible to use a mixture of several Lewis bases. However, one Lewis base alone is usually sufficient to be used in the reaction in every case. Potassium tert-butoxide is particularly preferred. In particular, it is commercially available as a solution in hydrocarbon media at a reasonable price and can be stored for long periods.

The solvent is a suitable solvent for the reaction that does not react with the starting and end products. These are, for example, inert solvents such as hydrocarbon solvents. Examples include: aprotic non-polar solvents, in particular tetrahydrofuran (THF), methyltetrahydrofuran (Me-THF), hexane, heptane, octane, nonane, decane, decalin, dichloroethane, toluene, xylene and methylene chloride. Mixtures of solvents can also be used.

It is self-explanatory that the reaction should be carried out anhydrous as far as possible (e.g. water content < 0.1 %), since the isocyanate and also the silyl azide would decompose in the presence of water.

The reaction is preferably carried out at a temperature of 0°C or less. For example, a temperature range of 0 to -80°C can be used. Lower temperatures (e.g. -90°C or less) or slightly higher temperatures (e.g. 1 to 5°C) may also be possible in individual cases.

The reaction time of the process according to the invention is extremely short. The process can be carried out almost quantitatively in a reaction time of a few seconds to a few minutes. For example, a reaction time in the range of 10 s to 10 min, preferably 30 s to 5 min, particularly preferably 30 s to 2 min, in particular 1 min.

This represents an enormous advantage over the known state-of-the-art processes, which emphasizes the great economic efficiency of the process.

The process of the invention has numerous advantages:
It provides a one-step synthetic method for carbamoyl azides, which allows the direct conversion of isocyanates into carbamoyl azides by reacting them with organic azides. This concept has not yet been described and allows the title compounds to be produced directly in a single synthetic step.

The reaction delivers the desired compounds under excellent chemical control. The starting compounds are readily available and can be handled without problems. The reactions are almost quantitative in all cases, which can eliminate the need for lengthy and expensive purification steps. This aspect makes the process extremely cost-efficient and sustainable, as the use of additional solvents, additional equipment and additional working hours to purify intermediate products or the end products is no longer necessary. This significantly increases the cost-effectiveness of the process and reduces the burden of additional waste materials.

The stereochemistry of the starting isocyanates is retained in the end product, which is a particularly great advantage, since the known syntheses for carbamoyl azides cannot do this.

The reaction time of the process according to the invention is extremely short and yet results in a yield of almost 100 %. This represents an enormous time saving compared to the known processes.

The following are examples of the process for preparing carbamoyl azides according to one embodiment of the invention, without limitation to the invention:

### Examples 1 to 27:

The process according to the invention was carried out according to the following reaction equations: 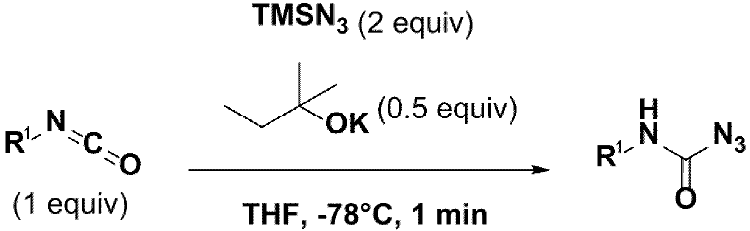 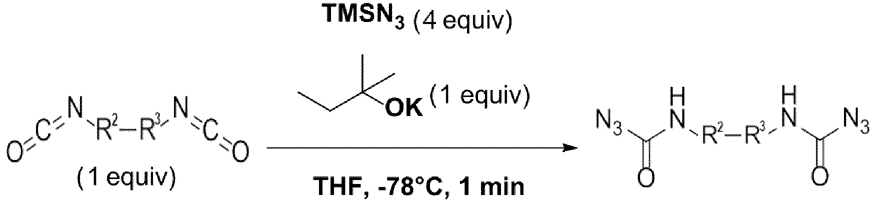 wherein R¹, R² and R³ are defined as previously indicated.

The process according to the examples was carried out as follows in accordance with the general synthesis procedure:
1 equivalent of the selected isocyanate was dissolved in dry tetrahydrofuran (THF) or dry methyl-tetrahydrofuran (Me-THF) at -78°C. Trimethylsilyl azide (TMSN₃, 2 equivalents) was added at the same temperature, followed by the addition of 0.5 equivalents of potassium tert-pentoxide (CH₃CH₂C(CH₃)₂OK). The reaction was quenched after only 1 minute at the same temperature. The crude product was then extracted with ethyl acetate, washed three times with saturated NaCl solution, dried over ammonium sulphate, filtered and concentrated under reduced pressure to obtain the corresponding final product (eventually) after chromatography on silica gel.

All reactions were monitored by ¹H NMR analysis. The compounds produced were of adequate analytical purity, as verified by full NMR characterization (¹H, ¹³C, possible heteronuclei), IR analysis and HRMS analysis. For optically active compounds - part of the optical rotation measurements - a chiral HPLC analysis was carried out in comparison to racemic mixtures. In addition, the melting point ranges for crystalline compounds are checked. Finally, an X-ray crystallographic analysis was carried out for selected compounds.

The above general synthesis procedure was used to produce the end products listed in the following table in the form of organic carbamoyl azides from the respective starting isocyanates:

**Table:**

| Example | starting compound | end product |
|---|---|---|
| 1 | 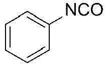 | 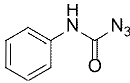 |
| 2 | 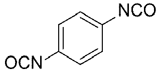 | 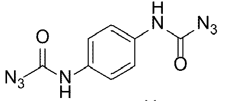 |
| 3 | 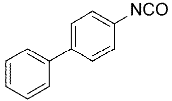 | 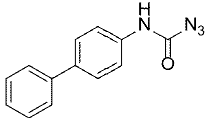 |
| 4 | 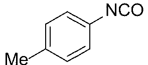 | 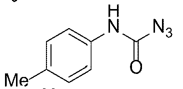 |
| 5 | 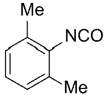 | 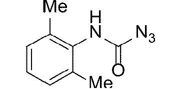 |
| 6 | 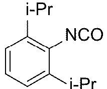 | 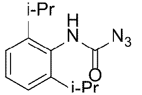 |
| 7 | 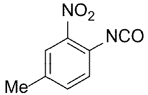 | 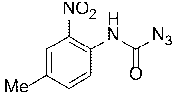 |
| 8 | 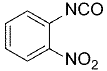 | 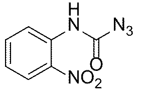 |
| 9 | 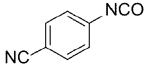 | 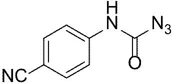 |
| 10 | 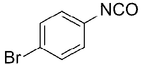 | 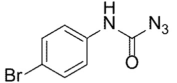 |
| 11 | 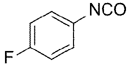 | 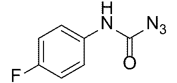 |
| 12 | 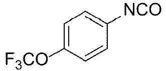 | 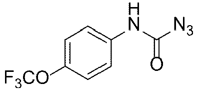 |
| 13 | 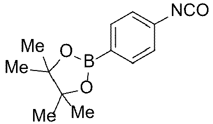 | 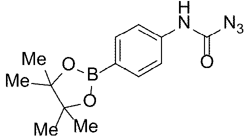 |
| 14 | 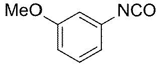 | 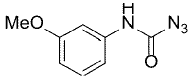 |
| 15 | 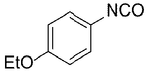 | 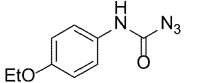 |
| 16 | 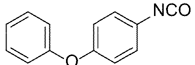 | 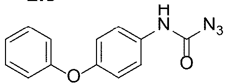 |
| 17 | 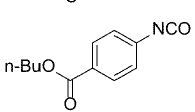 | 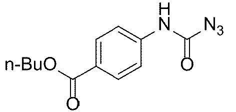 |
| 18 | 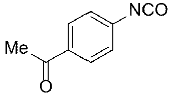 | 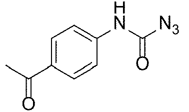 |
| 19 | 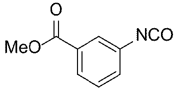 | 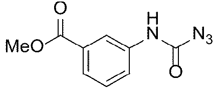 |
| 20 | 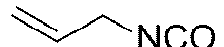 | 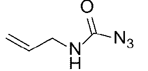 |
| 21 | 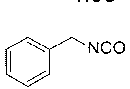 | 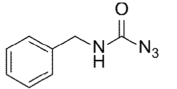 |
| 22 | 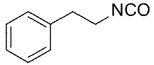 | 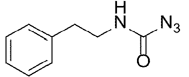 |
| 23 | 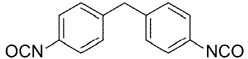 | 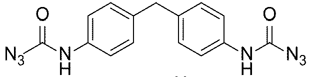 |
| 24 | 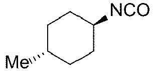 | 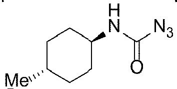 |
| 25 | 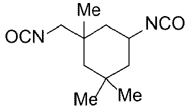 | 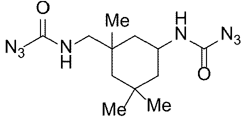 |
| 26 | 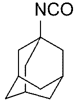 | 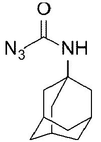 |
| 27 | 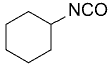 | 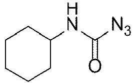 |

## Claims

1. A process for preparing a compound of general formula (I) 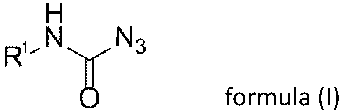
wherein R¹ is selected from the group consisting of
C₁-C₁₈-alkyl, which is optionally substituted with one or more substituents R⁴;
C₄-C₁₁-heterocyclyl, which is optionally substituted with one or more substituents R⁴;
C₂-C₁₈-alkenyl, which is optionally substituted with one or more substituents R⁴;
C₂-C₁₈-alkynyl, which is optionally substituted with one or more substituents R⁴;
C₆-C₁₈-aryl, which is optionally substituted with one or more substituents R⁴;
C₃-C₁₇-heteroaryl, which is optionally substituted with one or more substituents R4;
R⁴ is at each occurrence independently from another selected from the group consisting of:
NO₂,
CF₃,
CN,
OH,
=O,
OR⁵,
-(C=O)R⁵,
-(C=O)OR⁵,
halogen,
C₁-C₁₂-alkyl, which is optionally mono- or polysubstituted, the substituents being selected from the group consisting of NO₂, F₃CO, CF₃, CN, OH, =O, OR⁵, - (C=O)R⁵,
-(C=O)OR⁵, halogen;
C₄-C₁₁-heterocyclyl, which is optionally mono- or polysubstituted, the substituents being selected from the group consisting of NO₂, F₃CO, CF₃, CN, OH, =O, OR⁵, -(C=O)R⁵,
-(C=O)OR⁵, halogen;
C₂-C₁₂-alkenyl, which is optionally mono- or polysubstituted, the substituents being selected from the group consisting of C₆-C₁₀-aryl, NO₂, F₃CO, CF₃, CN, OH, =O, OR⁵, -(C=O)R⁵, -(C=O)OR⁵, halogen;
C₂-C₁₂-alkynyl, which is optionally mono- or polysubstituted, the substituents being selected from the group consisting of NO₂, F₃CO, CF₃, CN, OH, =O, OR⁵, - (C=O)R⁵,
-(C=O)OR⁵, halogen;
C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, OH, =O, OR⁵, -(C=O)R⁵, -(C=O)OR⁵, halogen, wherein C₁-C₅-alkyl is optionally substituted with one or more OH groups and C₂-C₈-alkenyl is optionally substituted with one or more OH groups;
C₃-C₁₇-heteroaryl, which is optionally mono- or polysubstituted, the substituents being selected from the group consisting of NO₂, F₃CO, CF₃, CN, OH, =O, OR⁵, -(C=O)R⁵,
-(C=O)OR⁵, halogen;
R⁵ is at each occurrence independently from another selected from the group consisting of:
CF₃,
C₁-C₁₂-alkyl, which is optionally mono- or polysubstituted, the substituents being selected from the group consisting of C₁-C₅-alkyl, NO₂, F₃CO, CF₃, CN, halogen; and
C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups and C₂-C₈-alkenyl is optionally substituted with one or more OH groups;
comprising the step of:
reacting a compound of general formula (II) 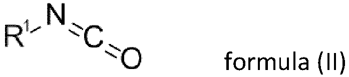
wherein R¹ is defined as hereinbefore,
with an organic silyl azide,
in a suitable solvent in the presence of a Lewis base.

2. The process according to claim 1,
**characterized in that**
instead of the compound of general formula (I), a compound of general formula (Ia) is prepared, 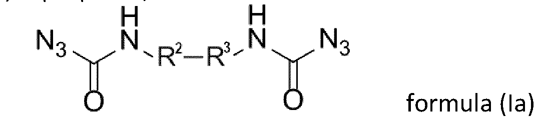
wherein R² and R³ are the same or different and are independently selected from the group as defined for R¹, whereby one of R² or R³ may also represent a direct bond,
by reacting a compound of general formula (Ila) 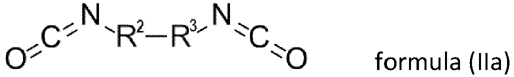
wherein R² and R³ are defined as hereinbefore,
with an organic silyl azide,
in a suitable solvent in the presence of a Lewis base.

3. The process according to claim 1 or 2,
**characterized in that**
the organic silyl azide is selected from the group consisting of alkyl silazide, aryl silazide or alkyl aryl silazide.

4. The process according to any one of claims 1 to 3,
**characterized in that**
the organic silyl azide is selected from a silyl azide having the general formula (III) 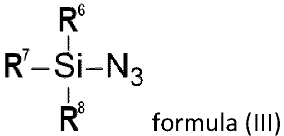
wherein R⁶, R⁷, and R⁸ are independently selected from the group consisting of
C₁-C₁₀-alkyl, which is optionally mono- or polysubstituted, the substituents being selected from the group consisting of C₁-C₅-alkyl, NO₂, F₃CO, CF₃, CN, halogen; and
C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups and C₂-C₈-alkenyl is optionally substituted with one or more OH groups.

5. The process according to any one of claims 1 to 4,
**characterized in that**
R⁶, R⁷, and R⁸ are independently selected from the group consisting of
C₁-C₅-alkyl, which is optionally mono- or polysubstituted, the substituents being selected from the group consisting of C₁-C₅-alkyl, NO₂, F₃CO, CF₃, CN, halogen; und
C₆-C₁₀-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups and C₂-C₈-alkenyl is optionally substituted with one or more OH groups.

6. The process according to any one of claims 1 to 5,
**characterized in that**
R⁶, R⁷, and R⁸ are independently selected from the group consisting of
C₁-C₅-alkyl, which is optionally mono- or polysubstituted, the substituents being selected from the group consisting of NO₂, F₃CO, CF₃, CN, halogen; and
phenyl, which is optionally mono- or polysubstituted, the substituents being selected from the group consisting of of C₁-C₅-alkyl, C₂-C₈-alkenyl, NO₂, F₃CO, CF₃, CN, halogen, wherein C₁-C₅-alkyl is optionally substituted with one or more OH groups and C₂-C₈-alkenyl is optionally substituted with one or more OH groups.

7. The process according to any one of claims 1 to 6,
**characterized in that**
R¹, R² and R³ are independently selected from the group consisting of
C₁-C₁₀-alkyl, which is optionally substituted with one or more substituents R⁴;
C₄-C₁₁-heterocyclyl, which is optionally substituted with one or more substituents R⁴;
C₂-C₈-alkenyl, which is optionally substituted with one or more substituents R⁴;
C₂-C₈-alkynyl, which is optionally substituted with one or more substituents R⁴;
C₆-C₁₄-aryl, which is optionally substituted with one or more substituents R⁴; and
C₃-C₁₇-heteroaryl, which is optionally substituted with one or more substituents R4.

8. The process according to any one of claims 1 to 7,
**characterized in that**
R¹, R² and R³ are independently selected from the group consisting of unsubstituted phenyl, phenyl substituted with one or more R⁴, unsubstituted naphthyl, naphthyl substituted with one or more R⁴, unsubstituted cyclohexane or cyclohexane substituted with one or more R⁴, whereby the substituents are selected from the group consisting of C₁-C₅-alkyl, C₂-C₈-alkenyl, NO₂, F₃CO, CF₃, CN or halogen, wherein C₁-C₅-alkyl is optionally substituted with one or more OH groups and C₂-C₈-alkenyl is optionally substituted with one or more OH groups.

9. The process according to any one of claims 1 to 8,
**characterized in that**
R¹ is selected from the group consisting of phenyl substituted with one or more R⁴, naphthyl substituted with one or more R⁴, or cyclohexane substituted with one or more R⁴, whereby the one or more R⁴ substituents are present in the ortho and/or para position to the isocyanate group of formula (II) or carbamoyl azide group of formula (I), where possible.

10. The process according to any one of claims 1 to 9,
**characterized in that**
R⁴ is at each occurrence independently from another selected from the group consisting of:
NO₂,
F₃CO,
CF₃,
CN,
OH,
=O,
OR⁵,
-(C=O)R⁵,
-(C=O)OR⁵,
halogen,
C₁-C₅-alkyl, which is optionally mono- or polysubstituted, the substituents being selected from the group consisting of NO₂, F₃CO, CF₃, CN, OH, =O, OR⁵, - (C=O)R⁵,
-(C=O)OR⁵, halogen;
C₄-C₁₁-heterocyclyl, which is optionally mono- or polysubstituted, the substituents being selected from the group consisting of NO₂, F₃CO, CF₃, CN, OH, =O, OR ⁵, -(C=O)R⁵, -(C=O)OR⁵, halogen;
C₂-C₈-alkenyl, which is optionally mono- or polysubstituted, the substituents being selected from the group consisting of C₆-C₁₀-aryl, NO₂, F₃CO, CF₃, CN, OH, =O, OR⁵, -(C=O)R⁵, -(C=O)OR⁵, halogen;
C₂-C₅-alkynyl, which is optionally mono- or polysubstituted, the substituents being selected from the group consisting of NO₂, F₃CO, CF₃, CN, OH, =O, OR⁵, - (C=O)R⁵,
-(C=O)OR⁵, halogen;
C₆-C₁₄-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, OH, =O, OR⁵, -(C=O)R⁵, -(C=O)OR⁵, halogen, wherein C₁-C₅-alkyl is optionally substituted with one or more OH groups and C₂-C₈-alkenyl is optionally substituted with one or more OH groups; and
C₃-C₁₇-heteroaryl, which is optionally mono- or polysubstituted, the substituents being selected from the group consisting of NO₂, F₃CO, CF₃, CN, OH, =O, OR⁵, -(C=O)R⁵, -(C=O)OR⁵, halogen.

11. The process according to any one of claims 1 to 10,
**characterized in that**
R⁵ is at each occurrence independently from another selected from the group consisting of:
CF₃,
C₁-C₁₀-alkyl, which is optionally mono- or polysubstituted, the substituents being selected from the group consisting of C₁-C₅-alkyl, NO₂, F₃CO, CF₃, CN, halogen;
C₆-C₁₄-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups and C₂-C₈-alkenyl is optionally substituted with one or more OH groups.

12. The process according to any one of claims 1 to 11,
**characterized in that**
the Lewis base is selected from the group consisting of tetrabutylammonium fluoride, cesium fluoride, potassium fluoride, potassium tert-butoxide, sodium methoxide and mixtures thereof.

13. The process according to any one of claims 1 to 12,
**characterized in that**
the solvent is selected from aprotic nonpolar solvents, in particular tetrahydrofuran (THF), methyltetrahydrofuran (Me-THF), hexane, heptane, octane, nonane, decane, decalin, dichloroethane, toluene, xylene and methylene chloride and mixtures thereof.

14. The process according to any one of claims 1 to 13,
**characterized in that**
the reaction is carried out at a temperature of 0°C or less, in particular in the range from 0 to -80 °C.

15. The process according to any one of claims 1 to 14,
**characterized in that**
the reaction is carried out over a period of a few seconds to a few minutes, in particular in the range of 10 s to 10 min, preferably 30 s to 5 min, particularly preferably 30 s to 2 min, in particular 1 min.

## Amended claims

### Amended claims in accordance with Rule 137(2) EPC.

1. A process for preparing a compound of general formula (I) 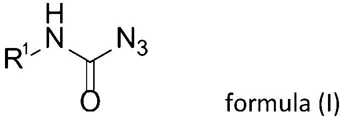
wherein R¹ is selected from the group consisting of
C₁-C₁₈-alkyl, which is optionally substituted with one or more substituents R⁴; C₄-C₁₁-heterocyclyl, which is optionally substituted with one or more substituents R⁴;
C₂-C₁₈-alkenyl, which is optionally substituted with one or more substituents R⁴; C₂-C₁₈-alkynyl, which is optionally substituted with one or more substituents R⁴; C₆-C₁₈-aryl, which is optionally substituted with one or more substituents R⁴; C₃-C₁₇-heteroaryl, which is optionally substituted with one or more substituents R⁴;
R⁴ is at each occurrence independently from another selected from the group consisting of:
NO₂,
CF₃,
CN,
OH,
=O,
OR⁵,
-(C=O)R⁵,
-(C=O)OR⁵,
halogen,
C₁-C₁₂-alkyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
C₄-C₁₁-heterocyclyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
C₂-C₁₂-alkenyl, which is optionally mono- or polysubstituted, the substituents being selected from the group consisting of C₆-C₁₀-aryl, NO₂, F₃CO, CF₃, CN, OH, =O, OR⁵, -(C=O)R⁵, -(C=O)OR⁵, halogen;
C₂-C₁₂-alkynyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, OH, =O, OR⁵, -(C=O)R⁵, -(C=O)OR⁵, halogen, wherein C₁-C₅-alkyl is optionally substituted with one or more OH groups and C₂-C₈-alkenyl is optionally substituted with one or more OH groups;
C₃-C₁₇-heteroaryl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
R⁵ is at each occurrence independently from another selected from the group consisting of:
CF₃,
C₁-C₁₂-alkyl, which is optionally mono- or polysubstituted, the substituents being selected from the group consisting of C₁-C₃-alkyl, NO₂, F₃CO, CF₃, CN, halogen; and
C₆-C₁₈-aryl, which is optionally mono- or polysubstituted, the substituents being selected from the group consisting of C₁-C₃-alkyl, C₂-C₈-alkenyl, C₂-C₃-alkynyl, C₆-C₁₀-aryl, NO₂, F₃CO, CF₃, CN, halogen, wherein C₁-C₃-alkyl is optionally substituted with one or more OH groups and C₂-C₈-alkenyl is optionally substituted with one or more OH groups;
whereby
the alkyl group comprises acyclic or cyclic, saturated, branched or linear hydrocarbon radicals,
the alkenyl group comprises linear, branched, and cyclic alkenyl substituents,
the alkynyl group comprises linear, branched, and cyclic alkynyl substituents, and
the alkoxy group comprises linear, branched, and cyclic alkoxy substituents;
comprising the step of:
reacting a compound of general formula (II) 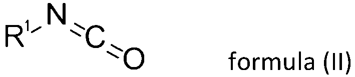
wherein R¹ is defined as hereinbefore,
with an organic silyl azide,
in a suitable solvent in the presence of a Lewis base.

2. A process for preparing ,a compound of general formula (la) 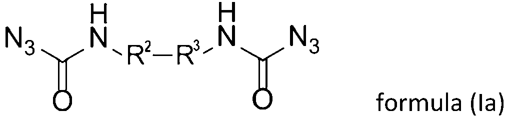 wherein R² and R³ are the same or different and are independently selected from the group as defined in claim 1 for R¹, whereby one of R² or R³ may also represent a direct bond,
by reacting a compound of general formula (Ila) 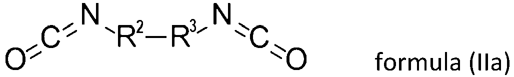
wherein R² and R³ are defined as hereinbefore,
with an organic silyl azide,
in a suitable solvent in the presence of a Lewis base.

3. The process according to claim 1 or 2,
**characterized in that**
the organic silyl azide is selected from the group consisting of alkyl silazide, aryl silazide or alkyl aryl silazide.

4. The process according to any one of claims 1 to 3,
**characterized in that**
the organic silyl azide is selected from a silyl azide having the general formula (III) 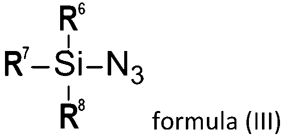
wherein R⁶, R⁷, and R⁸ are independently selected from the group consisting of C₁-C₁₀-alkyl, which is optionally mono- or polysubstituted, the substituents being selected from the group consisting of C₁-C₃-alkyl, NO₂, F₃CO, CF₃, CN, halogen; and
C₆-C₁₈-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups and C₂-C₈-alkenyl is optionally substituted with one or more OH groups.

5. The process according to any one of claims 1 to 4,
**characterized in that**
R⁶, R⁷, and R⁸ are independently selected from the group consisting of C₁-C₅-alkyl, which is optionally mono- or polysubstituted, the substituents being selected from the group consisting of C₁-C₅-alkyl, NO₂, F₃CO, CF₃, CN, halogen; und
Cₑ-C₁₀-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups and C₂-C₈-alkenyl is optionally substituted with one or more OH groups.

6. The process according to any one of claims 1 to 5,
**characterized in that**
R⁶, R⁷, and R⁸ are independently selected from the group consisting of
C₁-C₅-alkyl, which is optionally mono- or polysubstituted, the substituents being selected from the group consisting of NO₂, F₃CO, CF₃, CN, halogen; and phenyl, which is optionally mono- or polysubstituted, the substituents being selected from the group consisting of of C₁-C₅-alkyl, C₂-C₈-alkenyl, NO₂, F₃CO, CF₃, CN, halogen, wherein C₁-C₃-alkyl is optionally substituted with one or more OH groups and C₂-C₈-alkenyl is optionally substituted with one or more OH groups.

7. The process according to any one of claims 1 to 6,
**characterized in that**
R¹, R² and R³ are independently selected from the group consisting of C₁-C₁₀-alkyl, which is optionally substituted with one or more substituents R⁴;
C₄-C₁₁-heterocyclyl, which is optionally substituted with one or more substituents R⁴;
C₂-C₈-alkenyl, which is optionally substituted with one or more substituents R⁴;
C₂-C₈-alkynyl, which is optionally substituted with one or more substituents R⁴;
C₆-C₁₄-aryl, which is optionally substituted with one or more substituents R⁴; and
C₃-C₁₇-heteroaryl, which is optionally substituted with one or more substituents R⁴.

8. The process according to any one of claims 1 to 7,
**characterized in that**
R¹, R² and R³ are independently selected from the group consisting of unsubstituted phenyl, phenyl substituted with one or more R⁴, unsubstituted naphthyl, naphthyl substituted with one or more R⁴, unsubstituted cyclohexane or cyclohexane substituted with one or more R⁴, whereby the substituents are selected from the group consisting of C₁-C₅-alkyl, C₂-C₈-alkenyl, NO₂, F₃CO, CF₃, CN or halogen, wherein C₁-C₃-alkyl is optionally substituted with one or more OH groups and C₂-C₈-alkenyl is optionally substituted with one or more OH groups.

9. The process according to any one of claims 1 to 8,
**characterized in that**
R¹ is selected from the group consisting of phenyl substituted with one or more R⁴, naphthyl substituted with one or more R⁴, or cyclohexane substituted with one or more R⁴, whereby the one or more R⁴ substituents are present in the ortho and/or para position to the isocyanate group of formula (II) or carbamoyl azide group of formula (I), where possible.

10. The process according to any one of claims 1 to 9,
**characterized in that**
R⁴ is at each occurrence independently from another selected from the group consisting of:
NO₂,
F₃CO,
CF₃,
CN,
OH,
=O,
OR⁵,
-(C=O)R⁵,
-(C=O)OR⁵,
halogen,
C₁-C₅-alkyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
C₄-C₁₁-heterocyclyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
C₂-C₈-alkenyl, which is optionally mono- or polysubstituted, the substituents being selected from the group consisting of C₆-C₁₀-aryl, NO₂, F₃CO, CF₃, CN, OH, =O, OR⁵, -(C=O)R⁵, -(C=O)OR⁵, halogen;
C₂-C₅-alkynyl, which is optionally mono- or polysubstituted, the substituents being selected from the group consisting of NO₂, F₃CO, CF₃, CN, OH, =O, OR⁵, -(C=O)R⁵, -(C=O)OR⁵, halogen;
C₆-C₁₄-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, OH, =O, OR⁵, -(C=O)R⁵, -(C=O)OR⁵, halogen, wherein C₁-C₅-alkyl is optionally substituted with one or more OH groups and C₂-C₈-alkenyl is optionally substituted with one or more OH groups; and
C₃-C₁₇-heteroaryl, which is optionally mono- or polysubstituted, the substituents being selected from the group consisting of NO₂, F₃CO, CF₃, CN, OH, =O, OR⁵, -(C=O)R⁵, -(C=O)OR⁵, halogen.

11. The process according to any one of claims 1 to 10,
**characterized in that**
R⁵ is at each occurrence independently from another selected from the group consisting of:
CF₃,
C₁-C₁₀-alkyl, which is optionally mono- or polysubstituted, the substituents being selected from the group consisting of C₁-C₅-alkyl, NO₂, F₃CO, CF₃, CN, halogen;
C₆-C₁₄-aryl, which is optionally mono- or polysubstituted, the substituents being selected from the group consisting of C₁-C₅-alkyl, C₂-C₈-alkenyl, C₂-C₅-alkynyl, C₆-C₁₀-aryl, NO₂, F₃CO, CF₃, CN, halogen, wherein C₁-C₅-alkyl is optionally substituted with one or more OH groups and C₂-C₈-alkenyl is optionally substituted with one or more OH groups.

12. The process according to any one of claims 1 to 11,
**characterized in that**
the Lewis base is selected from the group consisting of tetrabutylammonium fluoride, cesium fluoride, potassium fluoride, potassium tert-butoxide, sodium methoxide and mixtures thereof.

13. The process according to any one of claims 1 to 12,
**characterized in that**
the solvent is selected from aprotic nonpolar solvents, in particular tetrahydrofuran (THF), methyltetrahydrofuran (Me-THF), hexane, heptane, octane, nonane, decane, decalin, dichloroethane, toluene, xylene and methylene chloride and mixtures thereof.

14. The process according to any one of claims 1 to 13,
**characterized in that**
the reaction is carried out at a temperature of 0°C or less, in particular in the range from 0 to -80°C.

15. The process according to any one of claims 1 to 14,
**characterized in that**
the reaction is carried out over a period of a few seconds to a few minutes, in particular in the range of 10 s to 10 min, preferably 30 s to 5 min, particularly preferably 30 s to 2 min, in particular 1 min.
